# EUROPEAN PATENT SPECIFICATION

(11) **EP 0 753 015 B1**
(45) Date of publication and mention of the grant of the patent: **16.08.2000**
(21) Application number: 95914366.0
(22) Date of filing: 29.03.1995
(51) Int. Cl.: C07K 16/40, C07K 16/28, C12N 5/20, G01N 33/577, G01N 33/573, G01N 33/574, A61K 51/10, A61K 39/395

(54) **MONOCLONAL ANTIBODIES RECOGNIZING TIE-RECEPTOR AND THEIR USE**
MONOKLONALE ANTIKÖRPER GEGEN DEN TIE-REZEPTOR UND DEREN VERWENDUNG
ANTICORPS MONOCLONAUX RECONNAISSANT LE RECEPTEUR "TIE", ET LEURS APPLICATIONS

(30) Priority: 29.03.1994 US 220240
(43) Date of publication of application: 15.01.1997
(73) Proprietor: Alitalo, Kari, 02100 Espoo (FI); Matikainen, Marja-Terttu, 23100 Mynämäki (FI); Karnani, Päivi, 20300 Turku (FI)
(72) Inventor: Alitalo, Kari, 02100 Espoo (FI); Matikainen, Marja-Terttu, 23100 Mynämäki (FI); Karnani, Päivi, 20300 Turku (FI)
(74) Representative: VOSSIUS & PARTNER
(86) International application number: PCT/FI95/00170
(87) International publication number: WO 95/26364

(56) References cited:
- WO-A-93/14124
- MOLECULAR AND CELLULAR BIOLOGY, vol. 12,no. 4, April 1992 WASHINGTON, DC, USA, pages 1698-1707, J. PARTANEN ET AL. 'A novel endothelial cell surface receptor tyrosine kinase with etracellular epidermal growth factor homology domains.' cited in the application
- PROTEIN ENGINEERING, vol. 4,no. 7, 1991 OXFORD, GB, pages 773-783, C. KETTLEBOROUGH ET AL. 'Humanization of a mouse monoclonal antibody by CDR-grafting: the importance of framework residues on loop confirmation.' cited in the application
- Kaipanen, A. et al (1994) Cancer Research 54: 6571-6577 Hatva, E. et al (1995) Amer. J. Pathol.146: 368-378

## Description

This invention relates to a specific antibody, which is reactive with Tie, a receptor tyrosine kinase found in various endothelial cells and in certain tumor cell populations. In addition, the present invention relates to methods of making said antibody and to methods of its use. Specifically, the present invention relates to an anti-Tie monoclonal antibody, as a diagnostic tool for detecting certain hematopoietic cells and hematological and angiogenesis associated conditions, as a tool for imaging blood vessels and as a therapeutic agent.

Cardiovascular diseases and cancer are very common in Western countries, and these disease groups are economically important because patients suffering from them typically stay out of work and need to be treated for prolonged periods. Blood vessels play an important role in the evolution of cardiovascular diseases, as well as in the pathogenesis of cancer. A central role in the pathogenesis of vascular diseases is played by endothelial cells lining the inner walls of blood vessels. Traumas and metabolic disturbances in endothelial cells give rise to so called atheroma plaques and further to arteriosclerosis. Neovascularization, induced by the tumor cells via growth factors stimulating endothelial cells, is an important event in various cancers. It is known from experimental investigations that in order to develop and grow colonies of cancer cells need neovascularization to ensure transport of nutrients and oxygen into the growing tissue.

The cellular behavior responsible for the development, maintenance, and repair of differentiated cells and tissues is regulated, in large part, by intercellular signals conveyed via growth factors and similar ligands and their receptors. The receptors are located on the surface of responding cells and they bind peptide or polypeptide growth factors, as well as other hormone-like ligands. As a result of this interaction rapid biochemical changes occur in the responding cells, which lead to both a rapid and a long-term readjustment of cellular gene expression. Several receptors associated with various cell surfaces may bind specific growth factors.

Tyrosine phosphorylation is one of the key modes of signal transduction across the plasma membrane. Several currently known protein tyrosine kinase genes encode transmembrane receptors for polypeptide growth factors and hormones, such as epidermal growth factor (EGF), insulin, insulin-like growth factor (IGF-I), platelet derived growth factors (PDGF-AA, AB and BB) and fibroblast growth factors (FGFs). See e.g., Heldin and Westermark, Cell Reg., 1:555-556 (1990); Ullrich and Schlessinger, Cell, 61:2243-354, (1990). Growth factor receptors of endothelial cells are of particular interest due to the possible involvement of growth factors, such as FGFs in several important physiological and pathological processes: angiogenesis, arteriosclerosis and inflammatory diseases (Folkman and Klagsbrun, Science, 235:442-447, 1987). Also, the receptors of several hematopoietic growth factors are tyrosine kinases. These include the colony stimulating factor 1 receptor (Sherr et al., Cell, 41:665-676, 1955) and c-kit, the stem cell factor receptor (Huang et al., Cell, 63:225-233, 1990).

The receptor tyrosine kinases can be divided into evolutionary subfamilies on basis of structural similarities and differences. These proteins differ in their specificity and affinity. (Ullrich and Schlessinger, supra). In general, receptor tyrosine kinases are glycoproteins, which consist of an extracellular domain, capable of binding the growth factor, a transmembrane domain, which usually is an alpha-helical portion of the protein, a juxtamembrane domain, where the receptor may be regulated by e.g. protein phosphorylation, a tyrosine kinase domain, which is the enzymatic component of the receptor and a carboxyterminal tail, which in many receptors is involved in recognition and binding of their specific substrates.

Recently, a novel endothelial cell receptor tyrosine kinase, designated Tie, has been described in International Patent Publication WO 93/14124. Tie is an acronym corresponding to **T**yrosine kinase containing **I**mmunoglobulin-and **E**GF-like domains. Tie is considered to be useful in the diagnosis and treatment of certain diseases involving endothelial cells and associated Tie-receptors, such as neoplastic diseases involving tumor angiogenesis, wound healing, thromboembolic diseases, atherosclerosis and inflammatory diseases.

The inventors have now produced monoclonal antibodies against the extracellular part of the endothelial cell receptor tyrosine kinase, Tie. These monoclonal antibodies have been used to detect Tie in cell cultures and by *in vivo* immunohistological tests. The results indicate that antibodies which specifically recognize the extracellular parts of Tie can be used for monitoring hematopoietic and endothelial cells in tissue samples and in an organism and for diagnosing various types of cancerous tumours.
Figure 1 represents an analysis of MOLT-4 and HEL cells by immunofluorescence for Tie and flow cytometry.
Figure 2 shows immunoperoxidase staining of Tie in human blood cells.
Figure 3 shows the biodistribution of ¹²⁵I-labelled monoclonal antibody 3C4C7G6 to selected target tissue in mice having an 8 day-old wound.
Figure 4. Immunohistochemical staining of Tie in normal brain, glioblastoma multiforme and melanoma metastasis. Scale bar: 0.05 mm.

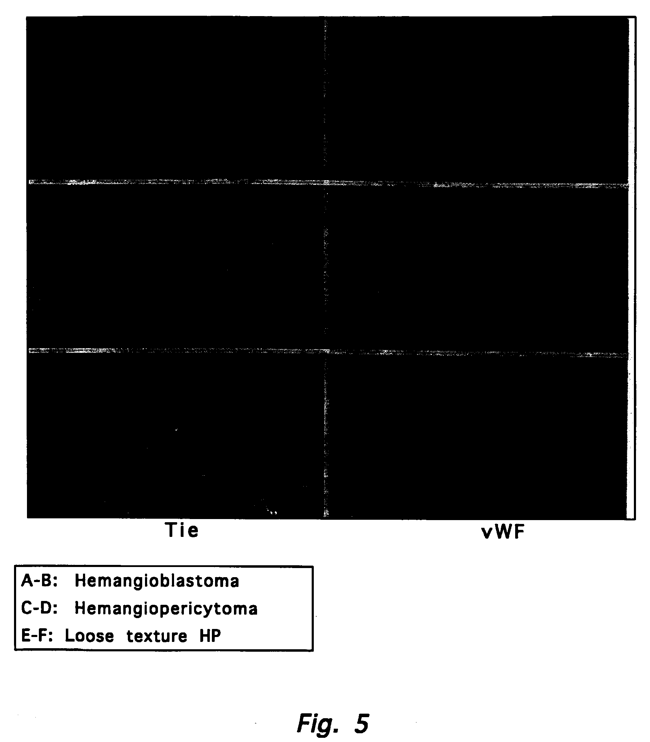

Figure 5. Immunohistochemical stains of hemangioblastoma and hemangiopericytoma with Tie and vWF or CD44 as endothelial cell-specific markers. (40x)
Figure 6. Distribution of anti-TIE-antibody 10F11 (%ID/g) in major organs at 48, 72, 96 and 120 hrs. (N = 3, 3, 3, and 4, respectively).
Figure 7. Distribution of anti-TIE-antibody 3c4 (%ID/g) in major organs at 6, 24, 48, 72, 96 and 120 hrs. (N = 3, 3, 7, 2, and 2 respectively).

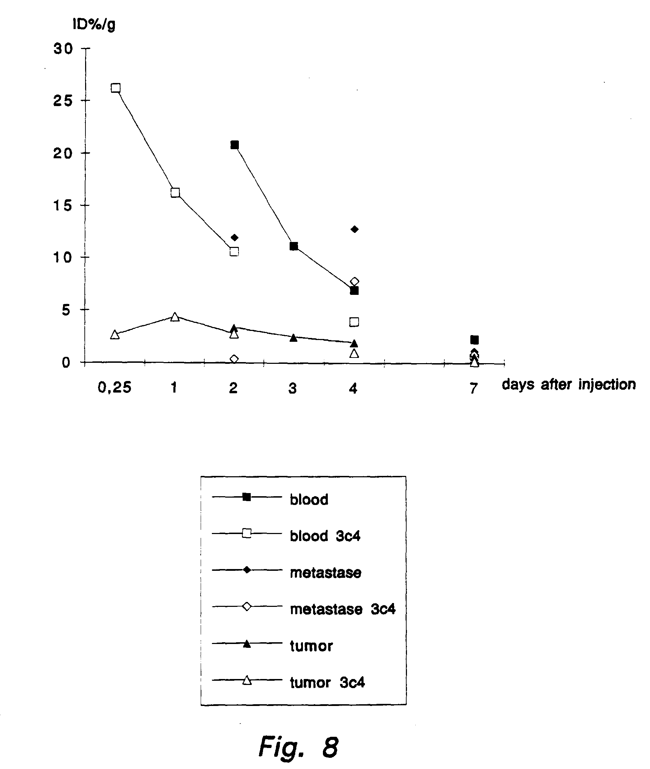

Figure 8. Accumulation of radioactivity at 48, 96 and 120hrs with antibody 10F11 and at 6, 24, 48, 96 and 120hrs with antibody 3c4.
Figure 9. Standard curve of four different pairs of coated antibody / labelled antibody.
Figure 10. Individual concentrations of Tie-antigen (µg/l) in serum samples of breast cancer (N=2), ovarian cancer (N=5) and small cell lung cancer (N=6) patients. Reference samples (N=20) are non-cancerous patients.

It is an object of this invention to provide diagnostic methods for monitoring hematopoietic and endothelial cells in tissue samples and in whole organisms. It is a further object of the present invention to provide clinical detection methods describing the state of endothelial cells (traumas, growth, etc.) and methods for detecting endothelial cells and thus vascular growth in an organism. The present invention provides an antibody recognizing Tie whereby this antibody is directed against extracellular portions of Tie.

More specifically, the invention provides a monoclonal antibody specifically recognizing an epitope of the extracellular parts of the Tie receptor. This monoclonal antibody is designated 3C4C7G6. The hybridoma cell line which produces monoclonal antibody 3C4C7G6 is deposited with the Deutsche Sammlung von Mikroorganismen und Zellkulturen GmbH (DSM) under the provisions of the Budapest Treaty (DSM accession number ACC2159).

The monoclonal antibody as mentioned above labelled with a detectable marker is also provided. As used herein, the term detectable marker encompasses any detectable marker known to those skilled in the art. However, in a preferred embodiment of this invention, the detectable marker is selected from the group consisting of radioisotopes, fluorochromes, dyes, enzymes and biotin. For the purpose of this invention suitable radioisotopes include, but are not limited to ¹²⁵I and ¹³¹I.

The present invention also provides the monoclonal antibody as mentioned above conjugated to an imageable agent. As used herein, the term imageable agent includes, but is not limited to, radioisotopes. A preferred radioisotope is 99m-technetium.

The present invention further provides a method for detecting and identifying human tissues undergoing neovascularization, which method comprises the steps of
(a) obtaining a tissue and/or body fluid sample suspected of undergoing neovascularization, and
(b) contacting said sample with the Tie-specific monoclonal antibody as mentioned above under conditions suitable for forming a complex between the monoclonal antibody and the antigen, and
(c) detecting the presence of any complex formed.

A tissue which may be detected by this method is any normal, precancerous or cancerous solid tumor tissue with Tie-containing endothelial cells or leukemia cells which express the Tie-receptor. In one embodiment of the present invention, the monoclonal antibody is labelled with a detectable marker as described herein. Methods of the invention are useful for detecting and differentiating various forms of cancer.

The present invention also provides a method for diagnosing and monitoring the disease state of various cancers, by determining the amount of circulating Tie-antigen in human serum obtained from a patient suspected of having a disease characterized by proliferation of endothelial cells.

The monoclonal antibody of the present invention may also be used in a method for detecting the presence of Tie-receptors in a cell sample, comprising the steps of exposing a cell sample to the monoclonal antibody of the present invention and detecting the binding of said monoclonal antibody to Tie-receptors.

In a preferred embodiment of the present invention the monoclonal antibody of the invention can be used to detect and monitor certain types of hematopoietic cells, especially cells in the B-cell lineage.

The exposure of a cell mixture to the monoclonal antibody of the invention can be in solution, as is the case for fluorescence-activated cell sorting, or it can be on solid tissue specimens, such as biopsy material, or it can be with the monoclonal antibody immobilized on a solid support, as is the case with column chromatography or direct immune adherence. The mixture of cells that is to be exposed to the monoclonal antibody can be any solution of blood cells or tissue cells. Prefereably, the cell mixture is from normal mammalian cells, mammalian bone marrow, circulating blood, or suspected tumor tissue, more preferably normal cells, leukemia cells and solid tumor cells. After exposure of the cell mixture to the monoclonal antibody, those cells with Tie -receptors will bind to the monoclonal antibody to form an antibody-Tie -receptor complex. The presence of the antibody-Tie -receptor complex, and therefore Tie receptors, can be detected by methods known in the art. These methods include ELISA, IRMA (a sandwich type of immunochemistry assay), immunohisto-chemistry, RIA using ¹²⁵I-label and autoradiography.

The invention also provides the use of the antibody of the present invention for the preparation of a composition for imaging the presence of angiogenesis in wound healing, in inflammation or in tumors of human patients, is also provided by this invention. This imaging comprises administration of labelled antibodies and detection by imaging at sites where endothelial cells are engaged in formation of new vessels or detection of leukemic cells in blood, bone marrow or tissues.

Humanized monoclonal antibodies of the present invention can be useful in treating neoplastic diseases involving endothelial cells with associated Tie receptors, by administration of a therapeutically effective amount of an anti-neoplastic therapeutic agent conjugated to such a monoclonal antibody to patients suffering from such diseases. A therapeutically effective amount of a therapeutic agent is any amount of a compound that will cause inhibition of growth of the tumor, preferably causing death of the neoplastic cells and a decrease in the total number of neoplastic cells in an organism. Examples of such therapeutic agents include antibodies coupled to the radioisotope 90Y or to toxin conjugates such as ricin and different microbial toxins.

Conjugation of the leukemia therapeutic agent to the monoclonal antibody can be accomplished using known techniques as described in e.g., Press et al., J. Clin.Oncol. 7:1027-1038 (1989). Preferably, the conjugation site on the monoclonal antibody is at a location distinct from the binding site for the monoclonal antibody to the Tie -receptor. It is also preferred that the conjugation site on the therapeutic agent be at a functional group distinct from the active site of the therapeutic agent. More preferably, the conjugation site will also be situated so as to minimize conformational changes of the monoclonal antibody or the therapeutic agent.

The present invention also relates to the use of a therapeutic agent conjugated to a binding fragment of the antibody of the present invention for the preparation of a pharmaceutical composition for treating neoplastic diseases whereby the treatment comprises the administration of a therapeutically effective amount of the therapeutic agent conjugated to the binding fragment of a monoclonal antibody of the present invention. Suitable binding fragments are those fragments that retain sufficient size and structure to allow binding of the fragment to the Tie-receptor. Such fragments can be prepared by numerous methods known in the art. The prepared binding fragments can be assayed for ability to bind to the Tie -receptor using the binding assays described in Example 5.

Administration of the monoclonal antibody of the present invention involves administration of an appropriate amount of a pharmaceutical composition containing the monoclonal antibody as an active ingredient. In addition to the active ingredient, the pharmaceutical composition may also include appropriate buffers, diluents and additives. Appropriate buffers include Tris-HCl, acetate, glycine and phosphate, prefereably phosphate at pH 6.5 to 7.5. Appropriate diluents include sterile aqueous solutions adjusted to isotonicity with NaCl, lactose or mannitol, preferably NaCl. Appropriate additives include albumin or helartin to prevent adsorption to surfaces, detergents (e.g., TWEEN 20™, TWEEN 80™), solubilizing agents (e.g., glycerol, plyethylene glycol), antioxidants (e.g., ascorbic acid, sodium metabisulfite) and preservatives (e.g., THIMERSOL™, benzyl alcohol, parabens).

Administration may be by any conventional means including intravenous, subcutaneous or intramuscular administration. The preferred route of administration is intravenous. Administration may be a single dose or may occur in an appropriate number of divided doses.

Preferably, the pharmaceutical preparation is in unit dosage form. In such form, the preparation is subdivided into unit doses containing the approriate quantities of the active component, e.g., an effective amount to achieve the desired purpose.

The actual dosage employed may be varied depending upon the requirements of the patient and the severity of the condition being treated. Determination of the proper dosage for a particular situation is within the skill of the art. Generally, treatment is initiated with smaller dosages which are less than the optimun dose of the compound. Thereafter, the dosage is increased by small increments until the optimum effect under the circumstances is administered essentially continuously or in portions during the day if desired. The amount and frequency of administration will be regulated according to the judgment of the attending clinician considering such factors as age, condition and size of the patient as well as severity of the disease being treated.

A typical recommended dosage regime for use in the present invention is from about 0.1 to about 10 mg active ingredient per day.

The development and use of mouse mAbs as therapeutic agents suffers from the fact, that the half life is reduced due to the formation of human antimouse antibody response (HAMA). Therefore the efficacy of the mouse monoclonal antibodies in patients is lower (review by Adair et al., 1990). Also adverse side-effects occur when repeated administrations of foreign proteins are used. Many of these problems can be solved using human monoclonal antibodies. At present these antibodies can be generated from mouse monoclonal antibodies using molecular biology techniques, where the complementary determining region (CDR) of mouse mAbs are joined with human mAbs. These humanized antibodies are suitable for use in immunotherapy in humans. Also single chain antibodies (scFv) will be constructed. In constructing these scFv's different lengths of linker sequences will be used as described by Whitlow et al. Protein Eng., 6(8): 989-95, (1993) in order to optimize the binding of the antibody to the antigen.

As is evident from the foregoing, antibodies according to the present invention are useful in the diagnosis and identification of disease states (e.g., various types of cancer), the detection and monitoring of wound healing, the treatment of various neoplastic diseases, and prophylaxis. Other uses of the presently-claimed subject matter are apparent to the skilled artisan.

The following examples are given to illustrate specific embodiments of the present invention, without limiting the scope therof. Other uses and embodiments of the present invention are readily appreciated by anyone skilled in the art.

### EXAMPLE 1

### Production of the extracellular domain of Tie in a baculovirus expression system

The cDNA sequence of the Tie protein has been disclosed in Partanen J., et al. *Mol*. *Cell Biol*. 12: 1698-1707, (1992). The cDNA sequence encoding the extracellular domain of Tie (amino acids 24-760) was PCR amplified and cloned into the BamHI site of pVT-Bac vector (Tessier et al., Gene, 98:177-1 83, 1991) using PCR primers
5'-CGTAGATCTGGCGGTGGACCTGAC-3' and
5'-GGCCATGATCACTAGTGATGGTGATGGTGATGCTGCTGATCCAGGCC CTCTTCAGC-3.
A sequence encoding a Factor X cleavage site (IEGA) followed by six consecutive histidine residues was inserted at the 3' end of the cDNA. The resulting vector, designated pVT-Tie, was then transfected into insect cells for expression of the Tie extracellular domain.

The pVT-Tie vector was cotransfected with Baculo Gold baculovirus DNA (Pharmingen Cat. 21100D) into SF-9 insect cells. Viral isolates were purified by plaque assay in agarose from the conditioned medium (TNMFH + 5%FCS) of the transfected cells and were tested for expression of the recombinant protein expression in High Five insect cells (Invitrogen). One of the isolates (BG-3 virus) was chosen for large scale protein production.

High Five cells were infected with the BG-3 virus and the conditioned medium (EX-CELL 400, JRH Scientific) of the infected cells was collected after two days. The recombinant BG-3 protein was purified from the medium by ConA affinity chromatography.

### EXAMPLE 2

### Production of anti-Tie monoclonal antibodies in Balb/C mice

Three months old Balb/c female mice were immunized by intraperitoneal injection of BG-3 (50 µg/mouse) emulsified with Freund's complete adjuvant. Booster injections of 50 µg were given at three-to-four week intervals and a final booster (20 µg BG-3 in PBS administered intravenously) after another three-week interval. Four days after the final booster dose, the mice were sacrified and mouse splenic lymphoid cells were fused with plasmacytoma cells SP 2/0 at a 2:1 ratio, respectively. The fused cells were harvested in 96-well culture plates (Nunc) in Ex-Cell 320 medium (Seralab) containing fetal calf serum (FCS, 20%) and HAT supplement (hypoxanthine- aminopterin-thymidin, Gibco, 043-01060H, diluted 50-fold). Cells were cultured at +37°C, in a 5% CO₂ atmosphere. After 10 days HAT-supplemented medium was changed to HT-supplemented cell culture medium (Gibco, 043-01065H, diluted 50-fold). HT medium was identical to HAT medium but without aminopterin.

Two to three weeks after fusion, specific antibody production was tested with by the antigen specific immunofluorimetric assay, IFMA, described in Example 5. The master clones were cloned by limited dilutions (Staszewski, 1984). Positive clones were expanded onto 24-well tissue culture plates (Nunc), recloned and retested by the same method. Positive clones were tested by fluorescence-activated cell sorting (FACS). The stable clone secreted immunoglobulins belonging to the IgG class.

One clone, designated 3C4C7G6 was found to stably secrete monoclonal antibody which was determined to be of immunoglobulin class IgG1 by IFMA. Hybridoma 3C4C7G6 was deposited with the German Collection of Microorganisms and Cell Cultures, Department of Human and Animal Cell Cultures and Viruses, Mascheroder Weg 1b, 3300 Braunschweig, Germany, December 2, 1993, and given accession No. ACC2159.

By similar methods other clones producing Tie-specific monoclonal antibodies directed against same or different epitopes were derived. One such clone 10F11G6 was chosen for further experiments together with the above disclosed 3C4C7G6.

Balb/c mice were used to produce monoclonal antibodies as ascites fluid. The hybridomas were intraperitoneally (i.p.) injected into mice after pretreatment of the animals with pristane (2,6,10,14-tetramethyl-pentadecan 98%, Aldrich-Chemie D-7924 Steinheim, cat.no T 2,280-2). 0.5 ml of pristane (i.p.) was injected about two weeks prior to the injection of the hybridoma cells. The amounts of cells injected were 7.5 to 9 x 10⁶ per mouse. The resultant ascites was collected 10-14 days after injection of the hybridomas, containing on average 0.3 mg/ml of antibody as determined by antigen specific IFMA as described in Example 6.

### EXAMPLE 3

### In-vitro production of anti-Tie monoclonal antibody in Hollow Fiber Bioreactors

Monoclonal antibodies against Tie were produced in vitro using the Tecnomouse System (Cellex Inc.). Media bottles with caps and filters were first autoclaved at 121°C and 1.1 bar pressure for half an hour. They were then filled with 1 L Dulbecco's MEM (Gibco, 042-02501, with glucose 6.4 g/L, glutamine 2 mmol/L 066-1051H, Na-pyruvate 1 mmol/L 066-1840E). The bioreactor holder was aseptically transferred in the Technomouse tray. The pump was loaded, and the medium lines as well as the empty waste bottles (the outflow line) aseptically connected.

The fill and flush programme was performed according to the manufacturer's instructions to wash all the preserving material from the Intracapillary space (IC) of the bioreactor. The program was started at a flow rate 150 ml/h for 4 hours. The washing was continued at flow rate of 50 ml/h for 20 hours with simultaneous washing of the Extracapillary (EC) space with 5% FCS in Dulbecco's MEM (DMEM). The medium in EC space was aseptically changed to fresh medium. One day later the Bioreactor was ready for inoculation of the hybridoma cells.

Hybridoma cells were harvested in cell culture bottles in 10% FCS - DMEM and 72 x 10⁶ cells were collected and inoculated in 5 ml volume of DMEM containing 5% FCS. The medium flowrate in the intracapillary space was 100 ml/h. The recycling method was used for harvesting monoclonal antibodies as follows: the medium line was connected to the medium bottle "out", taking the medium out from the bottle to the Bioreactor intra-capillary space; the outflow line was connected to the medium bottle "in" bringing the medium back to the bottle. Monoclonal antibodies were harvested three times a week on Monday, Wednesday and Friday, and a 10 ml volume of fresh medium containing 2.5% FCS in DMEM was replaced each time.

The anti-BG-3 cell line, 3C4C7G6, produced antibodies at mean concentration of 152 ug/ml in the cell culture medium. After inoculation of the cells to the Bioreactor in Tecnomouse system (72x10⁶ cells) the produced antibodies were harvested in two to three days period. The mean production was 4.5 mg/week and the cumulative production over 2 months was 37 mg.

The antibodies produced in either ascites fluid or in the Tecnomouse-system was purified by Affigel™ Protein A MAPS II Kit (BioRad) according to the manufacturer's instruction. The column was equilibrated for the purification procedure with binding buffer (pH 9.0). The antibodies were connected to the protein A -matrix in the binding buffer and washed with the binding buffer until a baseline was reached (detected at 280nm by UV-spectrometry). The specifically bound material was eluted from protein-A with elution buffer at pH 3.0 and the fractions were collected in the tubes containing the volume of 1 mol/L Tris-HCl pH 9.0, which was needed to neutralize the fraction immediately. Column was regenerated with regeneration buffer and stored till next use in 50 mmol/L Na-phosphate buffer pH 7.5 containing 0.05% NaN3 as preservative.

### EXAMPLE 4

### Production of antibodies against Tie expressed in bacteriae

A Bam HI fragment of Tie cDNA (nucleotides 520-1087) was subcloned into the Bam HI site of a pGEXIT vector (Pharmacia), resulting in an open reading frame encoding glutathione-S-transferase fused to a region encoding amino acids 162-350 of the Tie protein (GST-Tie2). The construct was transformed into an *E*. *coli* DH5a strain and expression of the fusion protein was induced by IPTG. The resulting 40 kD fusion protein was purified in a denaturing agarose gel (FMC) and used for immunizations.

Monoclonal antibodies were produced as described above for the BG-3 Tie protein. After two subclonings eight clones secreting antibodies against the GST-Tie2 protein were obtained. These reacted about equally well with Tie in Western immunoblotting. Ascites from anti GST-Tie2 clones was purified using protein-A column and used in Western blotting.

### EXAMPLE 5

### Labelling of Tie -protein with Europium

The extracellular domain of Tie produced in Example 1, BG-3, was labelled for use in assays. The labelling was performed according to Mukkala et al., *Anal*.*Biochem*. 176 (2):319-325, (1989), with modifications as follows: A 125 molar excess of isothiocyanate DTTA-Eu (N1 chelate, Wallac, Finland) was added to BG-3 solution (0.5 mg/ml in 50 mmol/L borate buffer, pH 8.6) and the pH was adjusted to 9.8 by adding one tenth of 0.5 mol/L sodium carbonate (Merck) buffer, pH 9.8. The labelling was performed overnight at +4°C. Unbound label was removed using PD-10 (Pharmacia, Sweden) with TSA buffer (50 mmol/L Tris-HCl pH 7.8 containing 0.15 mol/L NaCl) as eluent.

After purification 1 mg/ml bovine serum albumin (BSA) was added to the labelled BG-3 and the label was stored at +4°C.

The number of Europium ions incorporated per BG-3 molecule was 2.9, determined by measuring the fluorescence in ratio to that of known EuCl3 standards (Hemmilä et al., *Anal*.*Biochem*. 137: 335-343, 1984).

### EXAMPLE 6

### Immunofluorometric screening assay (IFMA)

Antibodies produced against the Tie receptor were screened using a sandwich-type immunofluorometric assay using microtiter strip wells (Nunc, polysorb) coated with rabbit antimouse Ig (Z 259, Dakopatts, Lovgren et al., Talanta 1984; 31 (10B): 909-916). The precoated wells were washed once by Platewash. 1296-024 (Wallac) with wash solution (DELFIA). The DELFIA assay buffer was used as a dilution buffer for cell culture supernatants and for serum of the splenectomized mouse (at dilutions between 1:1000 to 1:100,000) used as positive control in the preliminary screening assays.

AntiBG-3 3C4C7G6 produced as ascitic fluid and purified with Affigel™ Protein A MAPS was used as a standard in the later assays at a concentration between 0.25 ng/ml and 60 ng/ml in assay buffer (100 ul, DELFIA).

An incubation for 2 hours at room temperature (or alternatively an overnight incubation at +4°C) was begun by shaking on Plateshake (1296-001, Wallac) for 5 minutes followed by washing four times with wash solution as above.

The Eu-labelled BG-3 prepared in Example 4 was added at a concentration of 10 ng/well in 100 ul of the assay buffer. After 5 minutes on a Plateshake shaker and one hour incubation at room temperature, the strips were washed as described above.

Enhancement solution (DELFIA) was added at 200 ul/well. The plates were shaken for 5 minutes on Plateshake shaker and the intensity of fluorescence was measured by ARCUS-1230 (Wallac) in 10 to 15 minutes (Lövgren et al., In: Collins W.P. (ed), Alternative Immunoassays. John Wiley & Sons Ltd, 1985; 203-216).

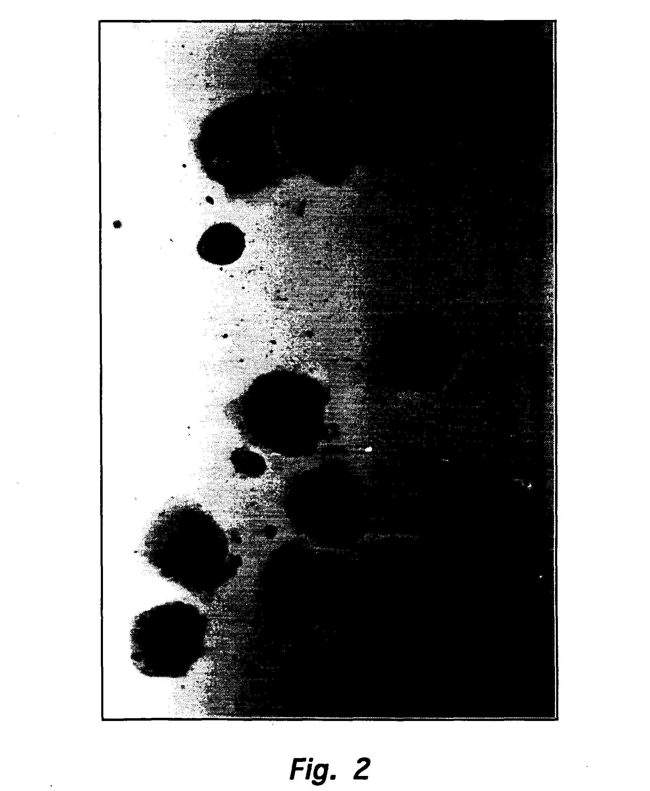

The sandwich-type DELFIA is very sensitive, the theoretical sensitivity being below 0.25 ng/ml for this anti-Tie monoclonal antibody. Although the sensitivity was convenient for quantitation of Mabs produced in cell culture supernatants the assay was also practical for quantitation of Mabs produced in vitro. The linear range reached from 0.25 ng/ml to 60 ng/ml (Figure 2). Intra assay variation was found to be very low.

### EXAMPLE 7

### Radiolabelled Monoclonal Antibody for in-vivo detection of Tie receptor

The monoclonal antibody 3C4C7G6 was labelled with ¹²⁵I using the chloramin-T method (Greenwood et at., *Biochem J*. 89:114-123, 1963). Na125I 1 mCi has been used to label 40ug antibody. Labelled antibody was purified by eluting with Sephadex-G25™ giving a main fraction of 1.8 ml.

I-125-labelled anti-BG-3 (3C4C7G6 ) was administered i.v. in two different doses of 1.2ug and 2.4ug to Lewis-lung-carcinoma bearing mice. The biodistribution of the labelled antibody in mice was measured at five different time points: 1) at 6h (N=4), 2) at 24h (N=7), 3) at 47h (N=5), 4) at 70h (N=3) and 5) at 117h (N=2).

**Table 1**

| Biodistribution to tissues at time points 1 - 5 | | | | | |
|---|---|---|---|---|---|
| | 1. | 2. | 3. | 4. | 5. |
| | % ID/g 6 h | % ID/g 24 h | % ID/g 47 h | % ID/g 70 h | % ID/g 117 h |
| blood | 36,22871 | 11,26086 | 9,343041 | 7,87285 | 2,749225 |
| hearth | 7,828641 | 2,842418 | 1,932395 | 1,391288 | 0,548053 |
| aortha | 16,07039 | 5,553582 | 3,707159 | 3,166192 | 0,798207 |
| lung | 9,091084 | 3,823566 | 3,087934 | 2,555693 | 0,987308 |
| liver | 6,940821 | 2,355467 | 1,770392 | 1,568976 | 0,685282 |
| kidney | 9,331627 | 3,490624 | 2,556933 | 1,829169 | 0,802883 |
| brain | 0,548253 | 0,228239 | 0,149502 | 0,146163 | 0,042287 |
| blood vess | 22,64432 | 4,712374 | 2,435758 | 2,65011 | 0,485038 |
| tumour | 7,151142 | 3,620555 | 3,155919 | 2,179194 | 0,901169 |
| spleen | 5,840843 | 2,004754 | 1,396837 | 1,195612 | 0,622342 |
| bladder | 6,889924 | 4,36051 | 3,628196 | 3,523912 | 0,899099 |
| ovarias | 8,445497 | 3,617958 | 2,622101 | 2,387713 | 0,662774 |

The results show that anti-BG-3 activity was concentrating to blood, tumor and blood vessels and in some amount to lungs and ovaries . Activity in blood was high at 48 and 70 hours time points: 9.3% ID/g and 7.9% ID/g (percentage of injected dose per gram of tissue normalized to a 20g mouse). Anti-Bg-3 activity in blood was 11.3% at 24 h and 36.2% at 6 hours time point.

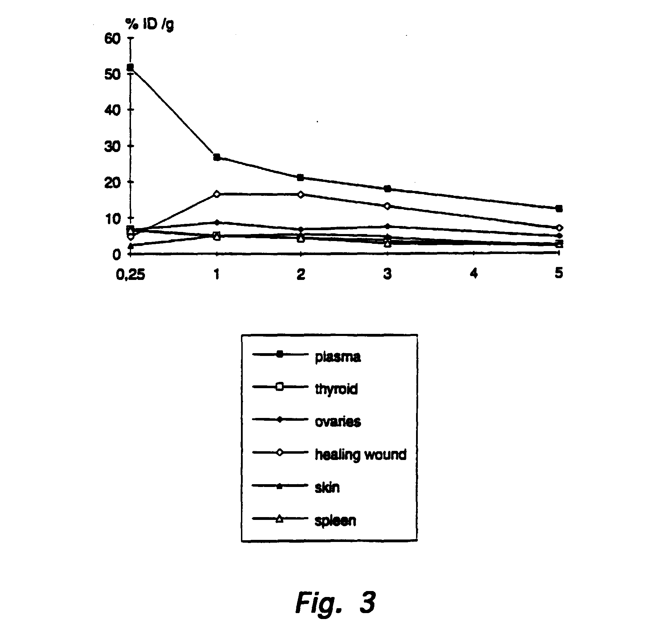

¹²⁵I-labelled anti-BG-3 (3C4C7G6) was also administered to mice having an 8 day-old wound in the skin epithelium ("wound healing mice"). The dose of antibody given to wound healing mice was 0.03 µg per animal. The biodistribution of antibody acitivity is shown in Figure 3. In that figure, the Y-axis represents the percent of injected dose (% ID/g) and the X-axis represents various time points of injection: 4h (N=2); 24h (N=6); 48h (N=6); 72h (N=4) and 120h (N=4). The equilibrium established between target and plasma is also shown in Figure 3.

### EXAMPLE 8

### Tc-99m-labelling of anti-Tie monoclonal antibodies for imaging studies

The antibody was labelled with tecnetium-99m using the technique of Schwarz et al., *J*.*Nucl*.*Med*., 1987, 28:721, and Mather et al., J.Nucl.Med., 1990, 31: 692-697. 2-Mercaptoethanol (ME) was used to open the disulphide bonds of the heavy chain in the hinge region of the immunoglobulin. Antibody was concentrated to approximately 10mg/L and sufficient ME added to the solution of antibody to provide a molar ratio of 1000:1 (0.47ul ME / 1mg antibody). The mixture was incubated at room temperature for 30 min and the reduced antibody purified by gel filtration on a 20ml Sephadex-G-50 column and eluted using phosphate-buffered saline as the mobile phase. The antibody fraction was pooled after measurement of optical density at 280 nm and stored at -20°C as 0.5 mg aliquots for labelling with 99m-Tc.

Upon labelling with 99m-Tc the antibody aliquot was thawed and reconstitued using a methylene diphosphonate (MDP) bone imaging kit (Amerscan Medronate II Technetium Bone Agent, N.165) with 5 ml 0.9% sterile saline according to the manufacturers instructions. 35 ul of the MDP solution, containing 35 ug MDP and 2.4 ug SnF₂, was added to antibody aliquot and mixed well. 99mTc pertechnetate was added to the mixture and shaken gently. The reaction was completed in 10 min. The radiochemical purity was measured by high pressure liquid chromatography.

Labelling of the reduced antibody gives a stabile 99m-Tc-labelled immunoglobulin because the unspecific binding of label is at minimum. The labelling efficiency is assessed by thin layer chromatography developed in 0.9% saline. The immunoreactivity is retained to not less than 85%. The in vivo stability will be analysed by cysteine challenge assay in vitro.

Anti-Tie-antibodies labelled with 99mTc can be detected with an ordinary gamma-camera or with SPECT (Single Photon Emission Computerized Tomography) to visualize the flow rate of antibody in a human body.

### EXAMPLE 9

### Detection of Tie-positive cell lines by FACS

The anti-Tie antibodies generated in Examples 2, 3 and 4 were used for detecting Tie protein in human leukemia cell lines.

The hematopoietic cell lines, HEL (human erythroleukemia) and MOLT-4 (T-lympholbastic leukemia) were obtained from ATCC. HEL-cells (Human ErytroLeukemia cells, which co-express erythroid and megakaryocyte markers were used for indirect immunofluoresence staining of Tie using the monoclonal antibodies generated and FACS analysis. Cells were counted, washed and incubated in the presence of several dilutions of the antibodies (from 1:1 to 1:200), washed and then incubated in the presence of FITC-conjugated antibodies against mouse immunoglobulins (secondary antibodies). Analysis was done by FACS IV. As a negative control the cells were stained with nonspecific mouse immunoglobulins, followed by the same secondary antibodies. As a negative cell control we used the MOLT4 T-cell leukemia line which does not express Tie mRNA.

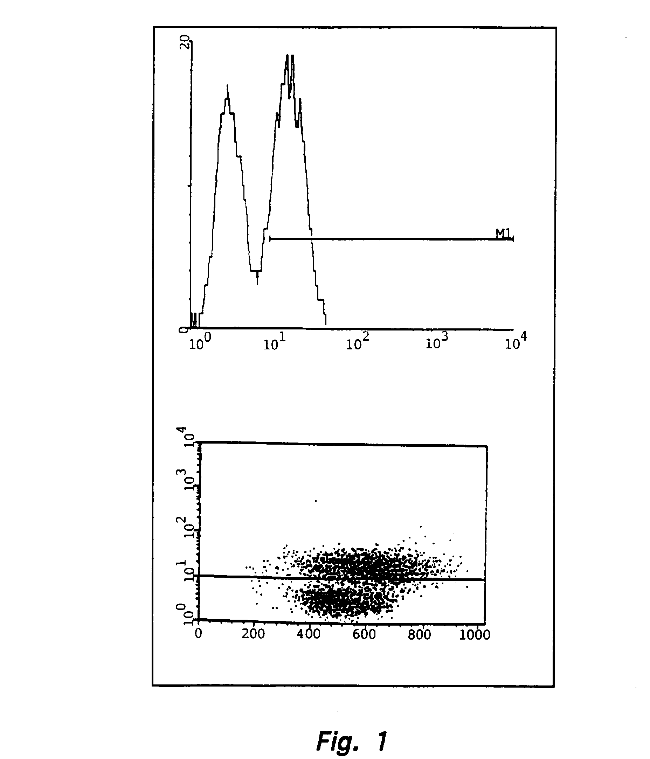

The results show that the anti-Tie antibodies stain on an average 85% of HEL cells while less than 1% of MOLT cells stain positively for Tie. When cells from these two lines are mixed the antibodies discriminate positive HEL cells from negative MOLT cells (Figure 1). From human bone marrow samples less than 1 % of cells stained also positive with these antibodies.

Cells from the human leukemia cell lines MOLT4 (a malignant T-cell line, which is Tie mRNA negative) and HEL (Human ErythroLeukemia cell line, Tie mRNA positive) were mixed in suspension at approximately 1:1 ratio. The cells were then stained in suspension using the monoclonal Tie antibodies derived in Example 2 diluted 1:10 and FITC conjugated anti-mouse IgG as the secondary antibody. As a negative control, the antibody was substituted with normal mouse serum. Analysis was done using FACS IV. The results indicate two distinct cell populations, one Tie positive the other Tie negative, each comprising about 50% of the whole cell population analyzed (Fig. 1).

Some of the Tie positive bone marrow cells were also positive for the CD19 B-call marker and all were negative for CD38. This shows that Tie is expressed in the B-cell lineage.

### EXAMPLE 10

### Analysis of the Monoclonal Antibodies

The monoclonal cell culture supernatants were tested for their ability to recognize Tie receptor on cell surfaces using FACS analysis. NIH3T3 cells transfected with Tie expression vector (full length Tie cDNA in pLTRpoly, Makela et al., 1991), control vector transfected NIH3T3 cells, as well as HEL cells (a human erythroleukemia cell line expressing endogenous Tie receptor) and MOLT-4 cells (a human T-cell leukemia cell line not expressing Tie) were incubated with conditioned medium of different cell clones followed by FITC labelled rabbit-anti mouse antibodies (Dako). The labelled cells were analysed by a fluorecence activated cell sorter (Beckton Dickinson).

Ascites was also produced from the following clones: 1H3H7, 3C4C7, 5C12H9. Clone 3C4D7 was further cloned and gave subclones 3C4C7G6, 3C4C7B11, 3C4C7E7, 3C4C7F9, which were similar to each other.

**Table 2**

| Results of anti-Tie monoclonal antibodies against the extracellular domain | | | | | |
|---|---|---|---|---|---|
| Clone | delfia | slot blot BG-3 | | Western | FACS |
| | | media | denat. | | |
| 1H3F10 | +++ | +++ | +++ | (+) | - |
| H6 | +++ | +++ | +++ | (+) | - |
| H7 | +++ | +++ | +++ | (+) | - |
| 3C4C7 | +++ | +++ | + | - | ++ |
| E4 | +++ | ++ | (+) | - | + |
| G4 | +++ | ++ | (+) | - | + |
| 5C12G11 | +++ | +++ | +++ | - | - |
| H9 | +++ | +++ | +++ | (+) | - |
| 6A11A11 | ++ | ++ | + | - | + |
| H6 | ++ | ++ | (+) | - | + |
| H9 | ++ | ++ | + | - | + |
| 9B10E6 | + | ++ | +++ | - | - |
| G7 | + | ++ | +++ | - | - |
| 9E7E9 | +++ | +++ | +++ | (+) | - |
| E10 | +++ | ++ | ++ | (+) | - |
| H6 | +++ | ++ | ++ | (+) | - |

### EXAMPLE 11

### Immunoperoxidase staining of Tie in human blood cells.

To mobilize hematopoietic stem cells into the peripheral blood, cyclophosphamide was given to a patient and buffy coat cells were collected from the peripheral blood seven days later. Red cells were hemolyzed from the cell suspension anc cytocentrigue slides prepared. These were then used for immunostaining using the purified Tie monoclonal antibody 3C4C7G6 and the immunoperoxidase method. In analysis of bone marrow cells by double immunofluorescence staining Tie positive cells (less than 1 %) could not be assigned to a clear hematopoietic lineage. Five positively staining cells were identified among the about 70,000 cells on a slide. In immunoperoxidase staining the Tie-positive cells were small and round, with a large nucleus and scant cytoplasm (Fig. 2). (The dark staining of one positive cell in the figure is the result of the peroxidase reaction and identifies a Tie-positive cell). Thus the anti-Tie monoclonal antibodies identify specific hematopoietic cells from the bone marrow and may be used for identification of defined subsets fo hematopoietic cells.

### EXAMPLE 12

### Humanization of Monoclonal Antibody 3C4C7G6

3C4C7G6 may be humanized using previously described methods (Kolbinger et al., 1993; Kettleborough et al., 1991). The humanization procedure involves incorporation of mouse kappa light (L) chain and heavy (H) chain complementarity determining regions (CDRs) into human variable (V) regions and making point mutations in human framework regions to preserve the original CDR conformations. The reshaped VL and VH chains will be joined to DNAs encoding human kappa and gamma-1 constant regions, respectively, in suitable expression vectors.

The generation of scFv is accomplished as previously described by Whitlow et al., *supra*, (1993).

The affinities of the humanized monoclonal antibodies and scFv's are tested using the methods described previously in this application.

### EXAMPLE 13

### Conjugation of Tie-specific Monoclonal Antibody to a Therapeutic Agent

Monoclonal antibody 3C4C7G6, or humanized antibodies as described in Example 12, may be coupled or conjugated to a variety of agents, for diagnostic use, as described in other examples herein, or for therapeutic use of the resulting conjugate.

For use in therapy of tumors and of dispersed malignancies such as leukemias, the antibodies may be coupled to radioisotopes such as ³²P, ¹³¹I, ¹²⁵I, ⁹⁰Y, ¹⁸⁸Re, ²¹²Pb, ²¹²Bi or ¹⁰B (See e.g., Scheinberg et al., Oncology, 1:31-37, 1987). Conjugation of radioisotopes to the antibody is accomplished by direct attachment of the isotopes to the antibodies, by methods described in the art (See e.g., Schwartz J., Nuclear Medicine 28:721, 1987) or by the aid of chelate linkers, which bind the radioisotope to the antibody or by a secondary antibody to the specific antibody. A variety of other agents may be attached to the antibodies. Such agents include antitumor drugs and antibiotics which are toxic by way of interaction with DNA via intercalation (e.g., daunomycin, adriamycin, aclacinomycin) or cleavage of DNA (e.g., esperamycin, calicheamycin, neocarzinostatin) and other toxic cytostatic drugs such as cis-platinum, vinblastine and methotrexate (see e.g., Greenfield et al., Antibody, Immunoconjucates and Radiopharmaceuticals, 4:107-119, 1991). These agents are coupled by covalent attachment of appropriate derivatives of the agents.

Many proteins and glycoproteins are also available for use in therapeutic conjugates of the antibodies. These include bacterial toxins such as Diptheria toxin, Shigella toxin and Pseudomonas exotoxin; plant toxins, such as ricin, abrin, modeccin, viscumin, pokeweed antiviral protein, saporin, momordin and gelonin. These toxins contain a catalytic fragment and in some cases fragments or domains that recognize cell surface structures or facilitate translocation across cell membrane. Appropriately modified toxins are used, which permit improved specificity without loss of potency. Conjugation of toxins to the antibodies is done by heterobifunctional crosslinkers, such as N-succinimidyl-3-(2-pyridyldithio)-propionate (SPDP) or 2-iminothiolane.

Prior to therapeutic use, conjugated antibodies are tested in view of their toxic potency, target specificity, in vitro and in-vivo stability and other properties (See e.g., Immunotoxins, Ed. Frankel, Kluwer Academic Publishers, Boston, 1988). It is desired that the toxicity of the conjugated agent, and the binding affinity and specificity of the antibody, is minimally affected by the coupling procedures used. The conjugates are therefore tested for binding to the Tie-receptor (see Example 10). In-vitro toxicity toward target cells such as the leukemia cell line Dami is tested by measuring incorporation of labeled compounds in treated versus control conjugate-treated cell cultures, and more directly by determining cultures that are able to grow in clonogenic and cell growth back-extrapolation assays. In-vivo stability, clearance, and specific toxicity are judged by administration of conjugates to appropriate animal recipients, such as mice, rats, rabbits or monkeys. Further such recipients include normal mice and in-vivo tumor and leukemia xenograft models comprising human neoplastic cells introduced into immunodeficient strains of mice, such as the nude mouse or SCID mouse.

### EXAMPLE 14

### Preparation of Pharmaceutical Composition Containing Monoclonal Antibody 3C4C7G6

Pharmaceutical compositions of the present invention include an effective amount of the active ingredient, a Tie-specific monoclonal antibody, especially monoclonal antibody 3C4C7G6, alone or in combination with a suitable buffer, diluent and/or additive. Such compositions are provided as sterile aqueous solutions or as lyophilized or otherwise dried formulations. Typically, antibodies are formulated in such vehicles at concentrations from about 1 mg/ml to 10 mg/ml.

One example of a suitable pharmaceutical composition for injection contains monoclonal antibody 3C4C7G6 (1 mg/ml) in a buffered solution (pH 7.0 +- 0.5) of monobasic sodium phosphate (0.45 mg/ml) and Tween 80™ (0.2 mg/ml) in sterile H2O. Pharmaceutical compositions according to the invention are administered in doses determined by the skilled artisan upon consideration of the targeted disease, severity of symptoms and characteristics of the patient. For example, pharmaceutical compositions of the invention may be applied locally to obtain maximum benefit in halting tumor growth and neovascularization or wound healing. However, different doses are necessary for systemic use, depending upon characteristics of the patient, such as weight, age, progression of disease, metabolism, and others.

Addititional embodiments will over to the skilled artisan upon consideration of the foregoing description. Accordingly, the present invention is limited only by the following claims.

### EXAMPLE 15

### Immunohistochemical staining of Tie in normal brain, glioblastoma multiforme and melanoma metastasis.

Fresh samples of previously untreated cerebral gliomas and intracranial meningeomas were obtained during open surgery at the Department of Neurosurgery, Helsinki University Central Hospital. All tumour patients were on corticosteroids. A sample of non-neoplastic control brain tissue was obtained during surgery for intractable epilepsy. Several of the tumour samples included adjacent brain tissue. All samples were frozen in liquid nitrogen as soon as possible after surgical removal and stored at -70°C. Histopathological diagnoses were based on haematoxylin/eosin stained cryostat sections as well as on routinely stained paraffin sections of adjacent formaldehyde-fixed tumour specimens. A complete list of the samples is shown in Table 3.

**Table 3**

| Human Tissue Specimens | | | |
|---|---|---|---|
| No | tissue samples | Age (y) | Sex |
| 1 | control brain | 66 | M |
| 2 | control brain | 11 | F |
| 3 | grade II oligoastrocytoma | 34 | F |
| 4 | grade II oligoastrocytoma | 66 | M |
| 5 | grade III astrocytoma | 36 | M |
| 6 | GBM | 53 | M |
| 7 | GBM | 63 | M |
| 8 | GBM | 56 | M |
| 9 | tissue adjacent to GBM | 69 | F |
| 10 | grade I meningeoma | 52 | M |
| 11 | grade II meningeoma | 35 | F |
| 12 | grade II meningeoma | 55 | M |
| 13 | melanoma metastasis | 54 | M |
| 14 | tissue adjacent to mm | 67 | M |
| 15 | grade III glioma | 44 | M |
| 16 | GBM | 65 | M |

| | | | |
|---|---|---|---|
| GBM = glioblastoma multiforme | | | |

Sterilised slides were dipped in 2% 3-aminopropyltriethoxysilane (TESPA) in acetone to ensure tissue adherence. 5-µm cryostat sections were cut at -20°C onto the pretreated slides, fixed in 4% paraformaldehyde and stored at -70°C.

5-µm cryostat sections from the samples used for *in situ* hybridisation analysis, as well as two additional malignant glioma samples, were cut onto TESPA slides as described previously. The sections were vacuum-dried at 37°C overnight and stained immunohistochemically using mouse monoclonal antibodies against human Tie (a cocktail of monoclonal antibodies against Tie extracellular domain) and rabbit antibodies (Dakopatts) against human von Willebrand factor (vWF) as an endothelial cell-specific marker.

Staining was carried out using the Vectastain ABC Elite biotin/avidin system for mouse IgG (Vector Laboratories, Burlingame, CA). Endogenous peroxidase activity in the dried, unfixed tissue sections was blocked by treatment with 0.5% H₂O₂ in methanol for 30 min. After PBS washes, the sections were incubated with diluted Vectastain blocking serum for 20 min and then with undiluted Tie antibody (1:1) overnight at 4°C or von Willebrand factor antibody (1:50) for 1 h at room temperature. Biotinylated secondary antibody was added for 45 min, followed by the Vectastain ABC reagent for 30 min. The reaction was visualised with 0.2 mg/ml 3-amino 9-ethylcarbazole (AEC), 0.03% H₂O₂, 14 mM acetic acid and 33 mM sodium acetate. Normal serum and antigen-blocked primary antibody were used as controls. Sections were lightly counterstained in haematoxylin and mounted in Aquamount.

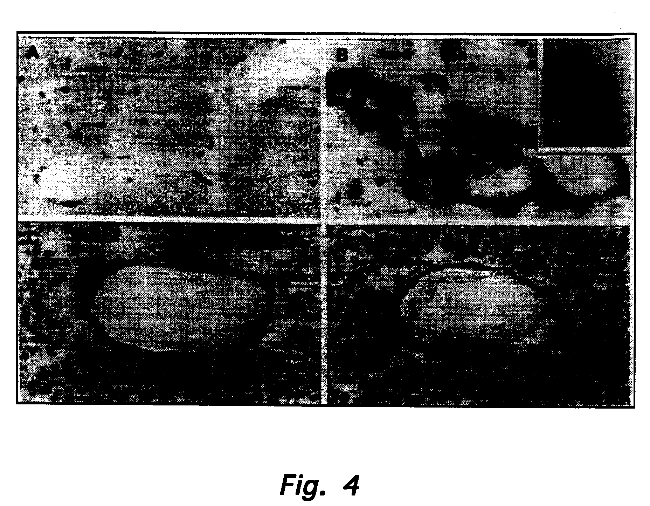

Blood vessels within normal cortical tissue were negative for Tie protein (Figure 4), whereas strong staining was observed in glioblastoma vasculature (B) and in the endothelia of tissue adjacent to GBM (inset, B). Tie protein was also detected in endothelial cells lining vessels in melanoma metastases (C) as well as bordering the tumour (data not shown). No specific staining was observed when the sections were incubated with antigen-blocked antibody or normal serum instead of the Tie antibodies. In order to further assess the extent and specificity of Tie staining, adjacent sections were stained for factor VIII (D). A summary of the Tie staining results is shown in Table 4.

**Table 4**

| Tie immunohistochemical staining | | |
|---|---|---|
| No | Tissue | Tie ab |
| 2 | normal brain | - |
| 15 | grade III glioma | ++ |
| 7 | GBM* | +++ |
| 16 | GBM | +++ |
| 11 | grade I meningeoma | ++ |
| 13 | melanoma metastases | ++ |

These results support a significant role for TIE in vascular angiogenesis accompanying tumour progression.

### EXAMPLE 16

### Immunohistochemical detection of the Tie protein in hemangioblastomas and hemangiopericytomas

In this study, we have analysed the expression of endothelial growth factors and their receptors in two highly vascular CNS tumours of controversial origin; capillary hemangioblastomas and hemangiopericytomas.

Fresh samples of hemangioblastomas and hemangiopericytomas were obtained during surgery at the Department of Neurosurgery, Helsinki University Central Hospital (All tumour patients were on corticosteroids) The samples were snap frozen immediately after surgical removal and stored at -70 °C. Histopathological diagnoses were based on haematoxylin/eosin stained cryostat sections and on routinely stained paraffin sections of adjacent formaldehyde-fixed tumour specimens.

Sterile slides were treated with 2% 3-aminopropyltriethoxysilane (TESPA) in acetone to ensure tissue adherence. 5-mm cryostat sections were cut onto the slides, fixed in 4% paraformaldehyde in PBS and stored at -70°C.

5-mm cryostat sections from the samples used for in situ hybridisation analysis, were cut onto TESPA slides as described previously. The sections were vacuum-dried at 37 °C overnight and stained immunohistochemically using mouse monoclonal antibodies against human Tie (monoclonal antibodies against Tie extracellular domain; a kind gift from Dr. Juha Partanen) and rabbit antibodies (Dakopatts) against human von Willebrand factor (vWF) and CD34 as an endothelial cell-specific markers (36).

Staining was carried out using the Vectastain ABC Elite biotin/avidin system for mouse IgG (Vector Laboratories, Burlingame, CA). Endogenous peroxidase activity in the dried, unfixed tissue sections was blocked by treatment with 0.5% H₂O₂ in methanol for 30 min. After PBS washes, the sections were incubated with diluted Vectastain blocking serum for 20 min and then with undiluted Tie antibody (1:1) overnight at 4 °C or CD34/von Willebrand factor antibody (1:50) for 1 h at room temperature. Biotinylated secondary antibody was added for 45 min, followed by the Vectastain ABC reagent for 30 min. The reaction was visualised with 0.2 mg/ml 3-amino 9-ethylcarbazole (AEC), 0.03% H₂O₂, 14 mM acetic acid and 33 mM sodium acetate. Normal serum and antigen-blocked primary antibody were used as controls. Sections were lightly counterstained in haematoxylin and mounted in Aquamount.

Strong expression of Tie protein was observed lining the inner wall of blood vessels in both hemangioblastomas (Figure 4, A,B) and, to a lesser extent, hemangiopericytomas (C-F). The levels of Tie protein varied substantially within the tumour tissues, for example, higher in a loosely textured hemangiopericytoma area (E, F), and lower in a more typical tumour sample (C, D). Overall, however, expression was significantly higher than that found in normal cortical specimens previousely analysed (23). In comparison with vWF immunostaining (B, D, F), Tie was not expressed by the hemangiopericytoma tumour cells (C-F).

These results are consistent with the hypothesis that Tie may play a role in angiogenesis in these two vascular tumours.

### EXAMPLE 17

### The in VIVO uptake of anti-TIE antibodies in Lewis lung carcinoma model in mouse

We have studied the uptake of radiolabeled (I¹²⁵) anti-TIE MoAbs (10F11G6 and 3C4C7G6) in the major organs of a mouse and in the lung metastases in order to determine the possibilities of using an anti-TIE MoAb in detecting malignant growth.

Radiolabelling of the antibodies was performed as follows: 40ug of antibody was labeled with 37MBq of Na-¹²⁵I using Chloramin-T method (Greenwood et al., *supra*, 1963). Labeled antibody was purified with Sephadex-G25 resulting to a main fraction of 1.8ml. Before in vivo biodistribution studies the affinity of three radiolabeled antibodies (3c4, 10F11, 7E8) was compared in a cell assay (Lindmo, Nucl Med 21: 807-810, 1984). All antibodies bind specifically to LE GD 14-2 cells, that are transfected mouse endothelial cells expressing the TIE receptor on their outer surface, but the binding of 10F11 was three times higher than the others (21 pMoles / 100 000 cells vs. 6-7 pMoles / 100 000 cells).

In lung carcinoma experiments we used 6- to 8- weeks old female mice (C57Bl/6, Bomholtgaard, Denmark) bearing Lewis lung carcinoma (LLC1/2) xenografts grown by injecting 1-10 M cells in 0.25ml per animal to right limb intramuscularly.

Thyroid blockade of mice was started one day before the injection of radiolabeled antibodies and continued through the experiment with KI solution (400mg/100ml) ad libidum.

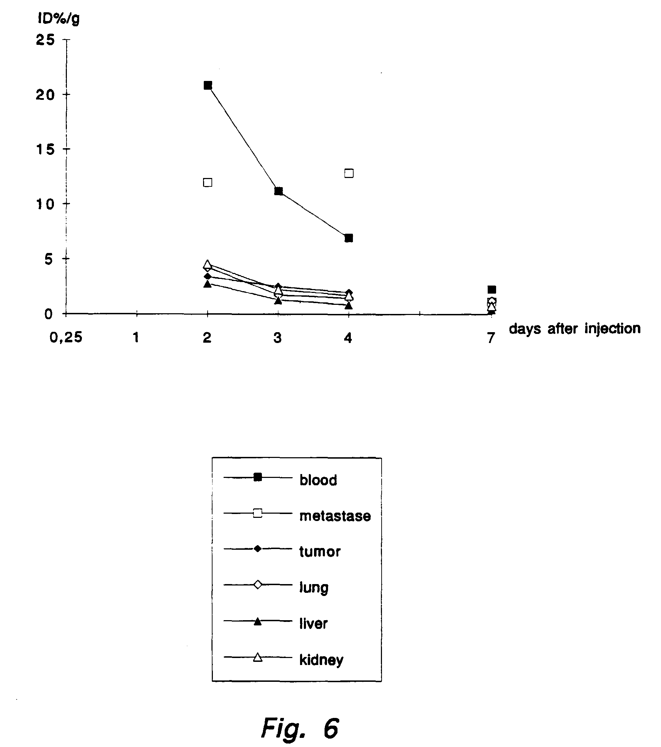

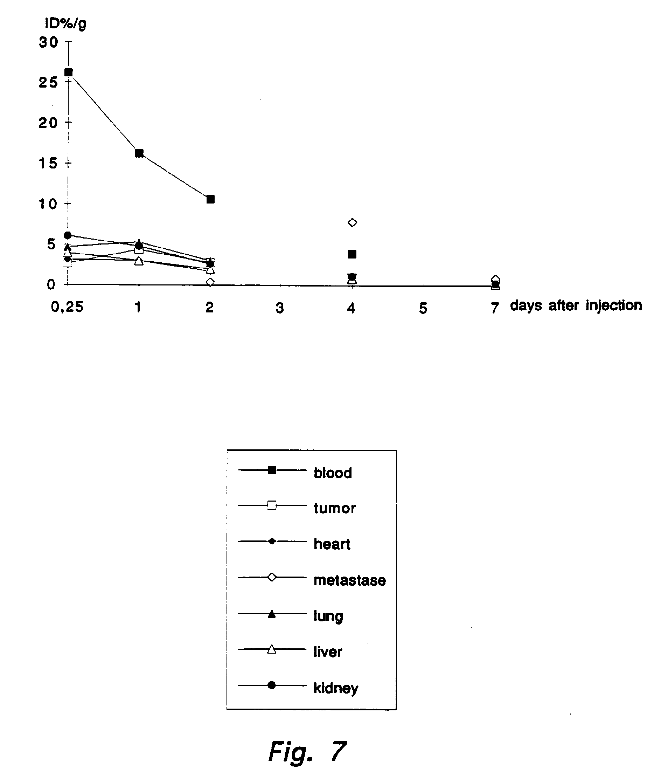

Biodistribution of I¹²⁵-anti-TIE antibodies was studied at 24, 48, 72, 96 and 120h after intravenous injection of 30ng antibodies in 20mM PBS. 14 animals having lung metastases of Lewis Lung ca have been studied with antibody 10F11: one at 24 hrs, three at 48 hrs, three at 72 hrs, three at 96 hrs and four at 7 days(see Fig. 6). Lung metastases have been present with antibody 3c4 only in three animals, at 24, 48 and 120hrs. (See Fig. 7).

3c4 is not showing binding to metastases in lung at 48 hrs time point (tissue-to-blood =0.07), but both are binding at 96hrs to the metastases of Lewis lung carcinoma model, tissue-to-blood ratios are 2.4 and 2.8 with 3C4C7G6 and 10F11G6, respectively.

The uptake of antibodies 3c4 and 10F11 in lung metastases is similar, both antibodies bind to metastases in vivo, but 10F11 is more strongly binding to serum: 20 %ID/g of 10F11 vs 10 %ID/g of 3c4 at 48

### EXAMPLE 18

### Serum sample analysis by IRMA assay

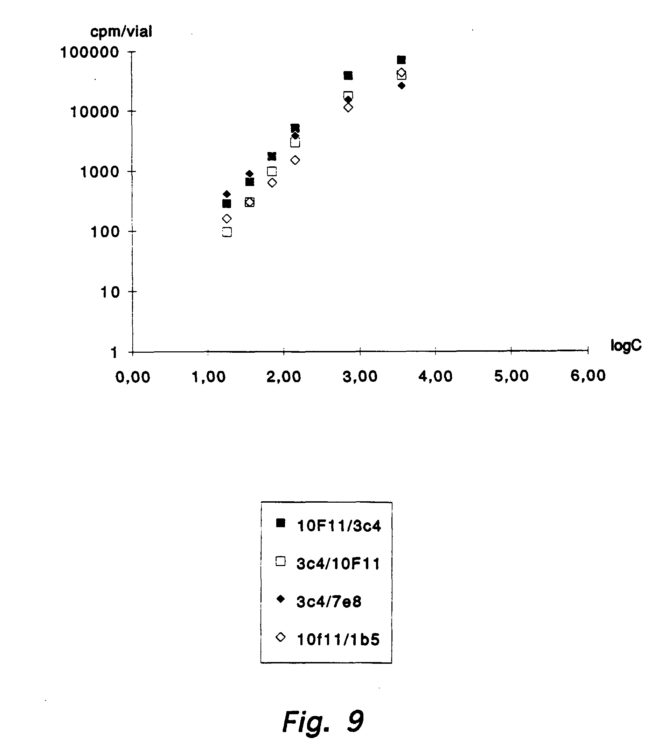

Serum samples of non-cancerous and cancer patients have been screened with a sandwich type of immunoradiometric (IRMA) assay (Miles L. et al., Nature 219:186-189(1968), where the catching anti-TIE-antibodies have been coated in polystyrene tupes and the second anti-TIE-antibodies have been radiolabelled with ¹²⁵I using the earlier mentioned chloramin-T-method. The recombinant-TIE-protein has been used as a standard preparation for estimation of the concentration of antigen in the serum samples . The concentration of the applied bulk standard preparation was measured using the absorbance on 280nm. A serial of dilutions of antigen was used to generate the standard curve, concentrations of tie-antigen were 0, 18, 36, 72, 144, 720 and 3600 µg/L. The standard curve of four different pairs of coated antibody / labelled antibody is presented in Figure 9.

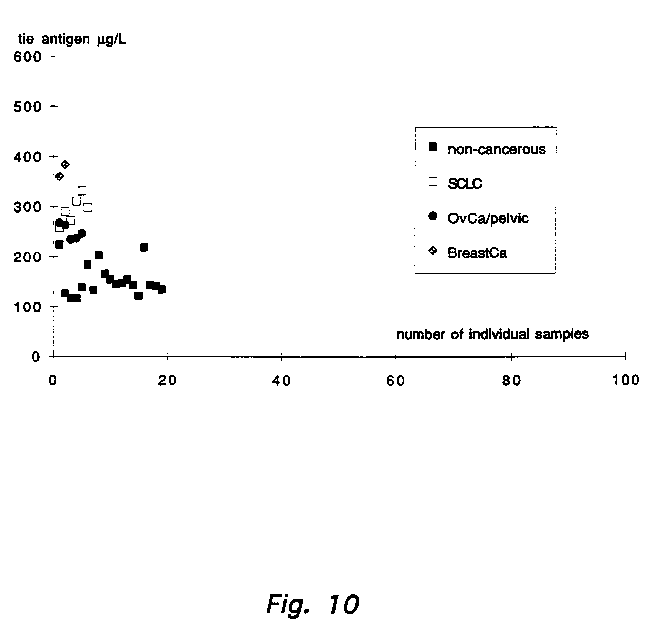

Serum samples of breast cancer, ovarian cancer and of different types of lung cancer patients have been screened with this assay as well as samples of non-cancerous patients. The patients who have had new metastases growing in lungs have shown elevated values in this assay. Patients with a stable disease show similar values than non-cancerous patients. The detected concentrations of the antigen in serum of the 20 non-cancerous patients and of three cancer patients that had fresh new metastases are shown in Figure 10. The patient with SCLC gave 6 samples, each one to two months appart and the patient with ovarian cancer + pelvic metastases gave 5 samples. The patient with breast cancer and a new lung metastases gave two samples. The pair of coated antibody / labelled antibody used in Fig. 10 was 3C4C7G6 / 10F11G6 that had better sensitivity according the standard curve (>5µg/L).

## Claims

1. A monoclonal antibody directed against the extracellular parts of a Tie-receptor tyrosine kinase wherein said antibody is anti-Tie monoclonal antibody 3C4C7G6 produced by the hybridoma cell line deposited as DSM accession number ACC2159.

2. A hybridoma cell line producing the monoclonal antibody according to claim 2 wherein said hybridoma cell line is deposited as DSM accession number ACC2159.

3. A detectably labelled antibody according to claim 1.

4. The detectably labelled antibody according to claim 3, wherein said detectable label is selected from the group consisting of radioisotopes, fluorochromes, dyes, enzymes and biotin.

5. A humanized monoclonal antibody according to claim 1, wherein a mouse light and heavy chain complementary-determining region of said monoclonal antibody is fused to a human variable region, and werein the conformation of said mouse complementary-determining region is preserved.

6. The monoclonal antibody according to claim 1 or 5, conjugated to a member of the group consisting of cytotoxic agents, cytostatic drugs and glycoproteins.

7. A method for detecting Tie in a biological sample comprising the steps of
a) exposing a sample suspected of containing Tie to the detectably labelled antibody directed against the extracellular part of Tie according to claim 3 or 4,
b) washing the sample; and
c) detecting the presence of said detectably labelled antibody in said sample.

8. The method according to claim 7, wherein the biological sample is selected from the group consisting of a solution containing blood cells or tissue cells or a solid tissue specimen.

9. The method according to claim 8, wherein the cells are selected from the group consisting of leukemia cells and bone marrow cells.

10. A method for diagnosing diseases characterized by proliferation of endothelial cells, comprising the steps of
a) obtaining a tissue sample from a patient suspected of having a disease characterized by proliferation of endothelial cells;
b) exposing said tissue sample to the detactably labelled antibody directed against the extracellular part of Tie according to claim 3 or 4;
c) washing said tissue sample; and
d) detecting the presence of said detectably labelled anti-Tie antibody in said tissue sample.

11. The method according to claim 10, wherein the disease is selected from the group of neoplastic diseases, such as leukemia, or diseases involving tumor angiogenesis, wound healing, thromboembolic diseases, atherosclerosis and inflammatory diseases.

12. The method according to claim 11, wherein the disease is selected from the group of megakaryoblastic leukemia, glioma, meningeoma, metastatic melanoma, hemangioblastoma, and hemangiopericytoma.

13. The use of the detectably labelled antibody directed against the extracellular part according to claim 3 or 4 for the preparation of a composition for imaging neovascularization in an organism.

14. The use according to claim 13, wherein said labelled antibody is labelled with 99m-technetium.

15. The use according to claim 13 or 14, wherein the imaging is performed by a gamma camera or with Single Photon Emission Computerized Tomography, SPECT.

16. A method for diagnosing and/or monitoring the disease state of cancer, comprising the steps of
a) obtaining a serum sample from a patient suspected of having a disease characterized by proliferation of endothelial cells;
b) exposing said serum sample to the detactably labelled antibody directed against the extracellular part of Tie according to claim 3 or 4;
c) detecting the presence of said detectably labelled anti-Tie antibody in said serum sample.

17. The method according to claim 16, wherein the disease state to be monitored is characterized by metastases.

18. The method according to claim 16 or 17, wherein the detection step is performed by a sandwich type assay.

19. A pharmaceutical composition comprising a therapeutically effective amount of the antibody directed against the extracellular part of Tie according to claim 1 or derivatives thereof in a pharmaceutically acceptable diluent, adjuvant or carrier.

20. The pharmaceutical composition according to claim 19, wherein said effective amount on an anti-Tie antibody is from about 1 mg/mi to about 10 mg/ml.

21. The use of the antibody directed against the extracellular part of Tie according to claim 1 for the preparation of a pharmaceutical composition for alleviating the symptoms of a disease characterized by abnormal growth of endothelial cells.

## Patentansprüche

1. Monoclonaler Antikörper, der gegen die extrazellulären Teile einer Tie-Rezeptor-Tyrosinkinase gerichtet ist, wobei der Antikörper der monoclonale Antikörper anti-Tie-3C4C7G6 ist, der von der Hybridomzellinie, die unter der DSM-Hinterlegungsnummer ACC2159 hinterlegt ist, produziert wird.

2. Hybridomzellinie, die den monoclonalen Antikörper nach Anspruch 1 produziert, wobei die Hybridomzellinie unter der DSM-Hinterlegungsnummer ACC2159 hinterlegt ist.

3. Nachweisbar markierter Antikörper nach Anspruch 1.

4. Nachweisbar markierter Antikörper nach Anspruch 3, wobei die nachweisbare Markierung aus der Gruppe bestehend aus Radioisotopen, Fluorochromen, Farbstoffen, Enzymen und Biotin ausgewählt ist.

5. Humanisierter monoclonaler Antikörper nach Anspruch 1, wobei eine komplementaritätsbestimmende Region der leichten und der schweren Kette des monoclonalen Maus-Antikörpers mit der variablen Region eines menschlichen Antikörpers fusioniert ist, und wobei die Konformation der komplementaritätsbestimmenden Region des Maus-Antikörpers erhalten ist.

6. Monoclonaler Antikörper nach Anspruch 1 oder 5, der mit einer Substanz ausgewählt aus der Gruppe bestehend aus cytotoxischen Agenzien, cytostatischen Arzneistoffen und Glycoproteinen konjugiert ist.

7. Verfahren zum Nachweis von Tie in einer biologischen Probe, umfassend die Schritte:
(a) Inkubation einer Probe, von der vermutet wird, daß sie Tie enthält, mit dem nachweisbar markierten Antikörper, der gegen den extrazellulären Teil von Tie gerichtet ist, nach Anspruch 3 oder 4;
(b) Waschen der Probe; und
(c) Nachweis des Vorhandenseins des nachweisbar markierten Antikörpers in der Probe.

8. Verfahren nach Anspruch 7, wobei die biologische Probe ausgewählt ist aus der Gruppe bestehend aus einer Lösung, die Blutzellen oder Gewebezellen enthält, und einer festen Gewebeprobe.

9. Verfahren nach Anspruch 8, wobei die Zellen ausgewählt sind aus der Gruppe bestehend aus Leukämiezellen und Knochenmarkszellen.

10. Verfahren zur Diagnose von Krankheiten, die durch die Proliferation von Endothel-Zellen gekennzeichnet sind, umfassend die Schritte:
(a) Erhalten einer Gewebeprobe von einem Patienten, bei dem eine Krankheit vermutet wird, die durch die Proliferation von Endothel-Zellen gekennzeichnet ist;
(b) Inkubation dieser Gewebeprobe mit dem nachweisbar markierten Antikörper, der gegen den extrazellulären Teil von Tie gerichtet ist, nach Anspruch 3 oder 4;
(c) Waschen der Gewebeprobe; und
(d) Nachweis des Vorhandenseins des nachweisbar markierten anti-Tie-Antikörpers in der Gewebeprobe.

11. Verfahren nach Anspruch 10, wobei die Krankheit ausgewählt ist aus der Gruppe bestehend aus neoplastischen Krankheiten wie Leukämie, Krankheiten, die Tumorangiogenese umfassen, Wundheilung, thromboembolische Krankheiten, Atherosklerose und entzündlichen Erkrankungen.

12. Verfahren nach Anspruch 11, wobei die Krankheit ausgewählt ist aus der Gruppe bestehend aus megakaryoblastischer Leukämie, Gliom, Meningeom, metastatischem Melanom, Hemangioblastom und Hemangiopericytom.

13. Verwendung des nachweisbar markierten Antikörpers, der gegen den extrazellulären Teil von Tie gerichtet ist, nach Anspruch 3 oder 4 zur Herstellung einer Zusammensetzung zur Abbildung von Gefäßneubildung in einem Organismus.

14. Verwendung nach Anspruch 13, wobei der markierte Antikörper mit 99m-Technetium markiert ist.

15. Verwendung nach Anspruch 13 und 14, wobei die Abbildung durch eine gamma-Kamera oder mit "Single Photon Emission Computerized Tomography" (SPECT) erfolgt.

16. Verfahren zur Diagnose und/oder Überwachung des Stadiums von Krebs, umfassend die Schritte:
(a) Erhalten einer Serumprobe von einem Patienten, bei dem eine Krankheit vermutet wird, die durch die Proliferation von Endothel-Zellen gekennzeichnet ist;
(b) Inkubation der Serumprobe mit dem nachweisbar markierten Antikörper, der gegen den extrazellulären Teil von Tie gerichtet ist, nach Anspruch 3 oder 4;
(c) Nachweis des Vorhandenseins des nachweisbar markierten anti-Tie-Antikörpers in der Serumprobe.

17. Verfahren nach Anspruch 16, wobei das zu überwachende Stadium durch Metastasen gekennzeichnet ist.

18. Verfahren nach Anspruch 16 oder 17, wobei der Nachweisschritt über einen "Sandwich"-Test erfolgt.

19. Arzneimittel umfassend eine therapeutisch wirksame Menge des Antikörpers, der gegen den extrazellulären Teil von Tie gerichtet ist, nach Anspruch 1 oder von Derivaten davon in einem pharmazeutisch verträglichen Verdünnungsmittel, Adjuvans oder Träger.

20. Arzneimittel nach Anspruch 19, wobei die wirksame Menge eines anti-Tie-Antikörpers in einem Bereich von etwa 1 mg/ml bis etwa 10 mg/ml liegt.

21. Verwendung des Antikörpers, der gegen den extrazellulären Teil von Tie gerichtet ist, nach Anspruch 1 zur Herstellung eines Arzneimittels zur Linderung der Symptome einer Krankheit, die durch das abnormale Wachstum von Endothel-Zellen gekennzeichnet ist.

## Revendications

1. Anticorps monoclonal dirigé contre les parties extracellulaires d'une tyrosinekinase récepteur Tie, dans lequel ledit anticorps est un anticorps monoclonal anti-Tie 3C4C7G6 produit par la lignée de cellules d'hybridome déposée sous le numéro d'enregistrement DSM ACC 2159.

2. Lignée de cellules d'hybridome produisant l'anticorps monoclonal selon la revendication 1 dans laquelle ladite lignée de cellules d'hybridome est déposée sous le numéro d'enregistrement DSM ACC 2159.

3. Anticorps à marquage détectable selon la revendication 1.

4. Anticorps à marquage détectable selon la revendication 3, dans lequel ledit marqueur détectable est choisi dans l'ensemble comprenant les radio-isotopes, les fluorochromes, les colorants, les enzymes et la biotine.

5. Anticorps monoclonal humanisé selon la revendication 1, dans lequel une région dudit anticorps monoclonal déterminant la complémentarité des chaînes légères et des chaînes lourdes de souris est fusionnée à une région variable humaine, et dans lequel la conformation de ladite région déterminant la complémentarité de souris est conservée.

6. Anticorps monoclonal selon la revendication 1 ou 5, conjugué à un membre du groupe comprenant les agents cytotoxiques, les médicaments cytostatiques et les glycoprotéines.

7. Procédé de détection du Tie dans un échantillon biologique, comprenant les étapes de
a) exposition d'un échantillon suspecté de contenir du Tie à un anticorps à marquage détectable dirigé contre la partie extracellulaire du Tie selon la revendication 3 ou 4,
b) lavage de l'échantillon ; et
c) détection de la présence, dans ledit échantillon, dudit anticorps à marquage détectable.

8. Procédé selon la revendication 7, dans lequel l'échantillon biologique est choisi dans l'ensemble comprenant une solution contenant des cellules sanguines ou des cellules de tissu ou un spécimen de tissu solide.

9. Procédé selon la revendication 8, dans lequel les cellules sont choisies dans l'ensemble comprenant les cellules de leucémie et les cellules de la moelle osseuse.

10. Procédé pour diagnostiquer des maladies caractérisées par une prolifération de cellules endothéliales, comprenant les étapes de
a) obtention d'un échantillon de tissu auprès d'un patient suspecté de présenter une maladie caractérisée par une prolifération des cellules endothéliales ;
b) exposition dudit échantillon de tissu à l'anticorps à marquage détectable dirigé contre la partie extracellulaire du Tie selon la revendication 3 ou 4,
c) lavage dudit échantillon de tissu ; et
d) détection de la présence, dans ledit échantillon de tissu, dudit anticorps anti-lie à marquage détectable.

11. Procédé selon la revendication 10, dans lequel la maladie est choisie dans l'ensemble comprenant les maladies néoplasiques telles que la leucémie, ou les maladies mettant en jeu une angiogenèse tumorale, une cicatrisation, des maladies thromboemboliques, une athérosclérose et des maladies inflammatoires.

12. Procédé selon la revendication 11, dans lequel la maladie est choisie dans l'ensemble comprenant la leucémie mégacaryoblastique, le gliome, le méningeome, le mélanome métastatique, l'hémangicblastome et l'hémangiopéricytome.

13. Utilisation d'un anticorps à marquage détectable dirigé contre la partie extracellulaire du Tie selon la revendication 3 ou 4 pour préparer une composition permettant l'imagerie d'une néovascularisation dans un organisme.

14. Utilisation selon la revendication 13, dans laquelle ledit anticorps marqué est marqué au 99m-technétium.

15. Utilisation selon la revendication 13 ou 14, dans laquelle l'imagerie est réalisée par une caméra gamma ou par une tomographie informatisée à émission d'un photon unique SPECT.

16. Procédé pour diagnostiquer et/ou suivre l'état pathologique d'un cancer, comprenant les étapes de
a) obtention d'un échantillon de sérum auprès d'un patient suspecté de présenter une maladie caractérisée par une prolifération de cellules endothéliales ;
b) exposition dudit échantillon de sérum à l'anticorps à marquage détectable dirigé contre la partie extracellulaire du Tie selon la revendication 3 ou 4 ;
c) détection de la présence dudit anticorps anti-Tie à marquage détectable dans ledit échantillon de sérum.

17. Procédé selon la revendication 16, dans lequel l'état pathologique à suivre est caractérisé par des métastases.

18. Procédé selon la revendication 16 ou 17, dans lequel l'étape de détection est réalisée par une analyse de type sandwich.

19. Composition pharmaceutique comprenant une quantité à effet thérapeutique de l'anticorps dirigé contre la partie extracellulaire du Tie selon la revendication 1 ou l'un de ses dérivés, dans un diluant, adjuvant ou excipient acceptable d'un point de vue pharmaceutique.

20. Composition pharmaceutique selon la revendication 19, dans laquelle ladite quantité efficace sur un anticorps anti-Tie est d'environ 1 à environ 10 mg/ml.

21. Utilisation de l'anticorps dirigé contre la partie extracellulaire du Tie selon la revendication 1 pour préparer une composition pharmaceutique destinée à soulager les symptômes d'une maladie caractérisée par une prolifération anormale de cellules endothéliales.
